Europäisches Patentamt

European Patent Office

Office européen des brevets

(11) Publication number: **0 517 361 A1**

# EUROPEAN PATENT APPLICATION

(21) Application number: **92303861.6**

(22) Date of filing: **29.04.92**

(51) Int. Cl.⁵: **C12Q 1/68**, //(C12Q1/68, C12R1:19),(C12Q1/68, C12R1:44),(C12Q1/68, C12R1:01)

(30) Priority: **30.05.91 US 707646**

(43) Date of publication of application:
**09.12.92 Bulletin 92/50**

(84) Designated Contracting States:
**DE FR GB IT**

(71) Applicant: **AMOCO CORPORATION**
**200 East Randolph Drive**
**Chicago Illinois 60601(US)**

(72) Inventor: **Weisburg, William Greene**
**3 Jillson Circle**
**Milford, MA 01757(US)**
Inventor: **Lane, David Joseph**
**9 Oriole Drive**
**Milford, MA 01757(US)**

(74) Representative: **Horton, Sophie Emma et al**
**Elkington and Fife Prospect House 8**
**Pembroke Road**
**Sevenoaks, Kent TN13 1XR(GB)**

(54) **A method for detecting and identifying pathogenic organisms using target sequences as detectors.**

(57) A method for detecting specific polynucleotide target sequences in samples is described. Oligonucleotide primers and deoxyribonucleotide triphosphate precursors, either of which are labeled, are combined with a polymerizing enzyme to create a labeled primer extension product which is complementary to the target polynucleotide sequence of interest. The replication cycle is repeated until there is sufficient primer extension product for detection. The labeled primer extension product is then captured by its hybridization to an immobilized complementary separation probe where it is easily detected.

FIG. 1A

## Background of the Invention

Antibody-based diagnostic procedures for the detection and identification of pathogenic microorganisms are currently one of the most rapid, convenient and inexpensive methods commercially available to clinical laboratories. Typically, these methods are based on detection of antibodies against the pathogen in patient samples or detection of a pathogen-specific protein directly, using antibodies specific for the protein. Nucleic acid based diagnostic procedures are based on detecting the presence of specific nucleotide sequences that are predetermined to be associated with particular disease states. These diagnostic procedures can be an earlier and a more specific predictor of disease than immunoassays. They are, however, complicated and time-consuming. There have been recent technological advances in this field, but nucleic acid based technology has not reached the level of ease that makes immunoassy based procedures commercially viable.

A more simplified and rapid nucleic-acid based assay procedure would be an important improvement in the technique available for the clinical diagnosis of infectious diseases.

## Summary of the Invention

The present invention relates to a method for the detection of an organism by detecting the presence in a sample of a target polynucleotide sequence which is contained within a single stranded polynucleotide or polynucleotides and is indicative of the organism. In one embodiment, the method involves detecting a target polynucleotide which is found in a broad spectrum of pathogenic bacteria: either ribosomal ribonucleic acid or a gene encoding the ribosomal ribonucleic acid which is indicative of the presence of a pathogen. The present method has two important advantages over available methods: it results in production of a target sequence or sequences which carry a detectable moiety and it provides for separation and detection of target sequence(s) by reliable, simple procedures. In the present method, the target sequence is amplified or polymerized, the polymerization product is detectably labeled as it is being produced and the resulting detectably labeled amplified target is readily separated from the sample. This is done through the use of two types of oligonucleotide sequences: 1) oligonucleotide primers, which are selected to hybridize to a target sequence present in a group of organisms (e.g., all bacteria, all bacteria of a particular genus or genuses), are contacted with a sample and serve as primers in oligonucleotide primed polymerization and 2) separation probes, which are bound to a solid support and may be generic separation probes (i.e., they hybridize to a sequence presenting a group of organisms) or specific separation probes (i.e., they hybridize to a sequence present in a particular organism or smaller group of organisms than that to which the oligonucleotide primer used hybridizes). Oligonucleotide primers, oligonucleotide precursors or both which are used in the polymerization step of the present method are detectably labeled and, thus, products of the oligonucleotide primed polymerization are detectably labeled.

In the present method, polynucleotides in a sample to be analyzed are rendered available for primer-extension (e.g., by releasing them from cells and, if necessary, rendering them single stranded). The polynucleotides are combined with at least one, and generally two, oligonucleotide primers, which are substantially complementary to the target polynucleotide sequence; deoxynucleotide triphosphate precursors and a polymerizing agent. These reagents are combined simultaneously or sequentially.

The resulting combination is maintained under conditions appropriate for hybridization of sufficiently complementary sequences, followed by oligonucleotide primed polymerization. This results in initial formation of primer/target polynucleotide complexes, followed by replication or polymerization of the target polynucleotide. The primer/target polynucleotide complexes, precursors (deoxynucleotide triphosphates or dNTPs) and polymerizing agent are maintained under appropriate conditions and for a sufficient time to complete one cycle of oligonucleotide primed polymerization. The polymerizing agent catalyzes the polymerization of the precursors and oligonucleotide primers, thereby creating a primer extension product. Completion of one cycle of polymerization results in production of a primer extension product/target polynucleotide complex in which the primer extension product is complementary to or substantially complementary to the target polynucleotide. At least one of the primers and/or precursors is labeled and at the completion of one cycle, the primer extension product is a labeled complement of the target polynucleotide sequence.

Subsequently, the labeled primer extension product/polynucleotide target complex is denatured and the replication cycle is continued until a sufficient number of labeled primer extension products are manufactured. After the initial cycle has been completed, the products of denaturation of the complex are the labeled primer extension product and the target polynucleotide. If more than one cycle of oligonucleotide primed polymerization is carried out, the resulting product is a combination of two types of complexes: labeled primer extension product/target polynucleotide complexes and labeled primer extension product/labeled primer extension product complement complexes (in which the primer extension product complement is substantially homologous to or homologous to the target polynucleotide). Thus, after second and subsequent cycles have been completed, the products of denaturation include the target polynucleotide, labeled primer extension product and labeled primer extension product complement, which is substantially homologous to the target polynucleotide. Because the primers and/or precursors are labeled, the resulting products are labeled. After each replication cycle, the resulting complexes are denatured to produce single-stranded polynucleotides. In subsequent replication cycles, the oligonucleotide primers used are substantially complementary to the target polynucleotide sequence or to the primer extension product.

The mixture from denaturation of the complexes is contacted with a set of separation probes immobilized on a solid support. The separation probes are complementary to a sequence, present in a labeled primer extension product or a sequence present in the labeled primer extension product complement. As a result, they hybridize, respectively, to labeled primer extension product and to labeled primer extension product complement. For convenience, both of these are referred to herein as labeled primer extension products. The separation probes may be selected to hybridize to a broad spectrum of pathogenic organisms, as were the primers (e.g., to all eubacteria or to all members of a bacterial genus), or to a subgroup or subgroups (e.g., a species) of pathogenic organisms. For example, a pair of oligonucleotides specific for hybridization to essentially all eubacteria (e.g., Probe 1740 and the complement of Probe 1660; described in U.S.S.N. 359,158, Universal Eubacteria Nucleic Acid Probes and Methods and corresponding Patent Cooperation Treaty application PCT/WO90/15157) can be used as the primers in the oligonucleotide primed polymerization step(s) and one or more oligonucleotides specific for one or more candidate bacterial species (e.g., oligonucleotides specific for sequences in 16S rRNA) can be used as the separation probes.

The immobilized separation probe(s) is reacted with a mixture containing products of oligonucleotide primed polymerization carried out as described under conditions appropriate for hybridization of complementary sequences to occur, thereby forming labeled primer extension product/separation probe/solid support complexes and labeled primer extension product complement/separation probe/solid support complexes. Once the primer extension product is bound, it is separated from other unbound nucleic acids by separating solid support from the sample. Once bound to the solid support, the labeled primer extension product is easily detected and its presence is indicative of the presence in the sample of the polynucleotide target sequence and associated organism.

The present invention has many advantages. The primer-extension step allows for the rapid increase of target material while simultaneously allowing for the incorporation of the detection moiety in the primer-extension product. This step allows for the elimination of time-consuming culturing steps, allowing for rapid assay times as well as a sufficient quantity of target material for multiple tests. In addition, the need for a labeled detection probe is eliminated and the primer extension product can be tested against a battery of different probes.

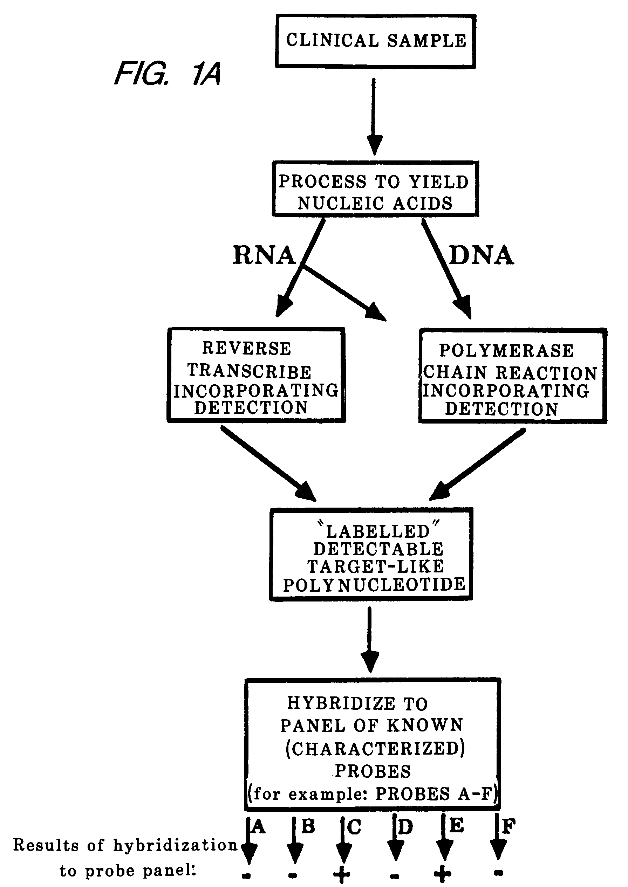

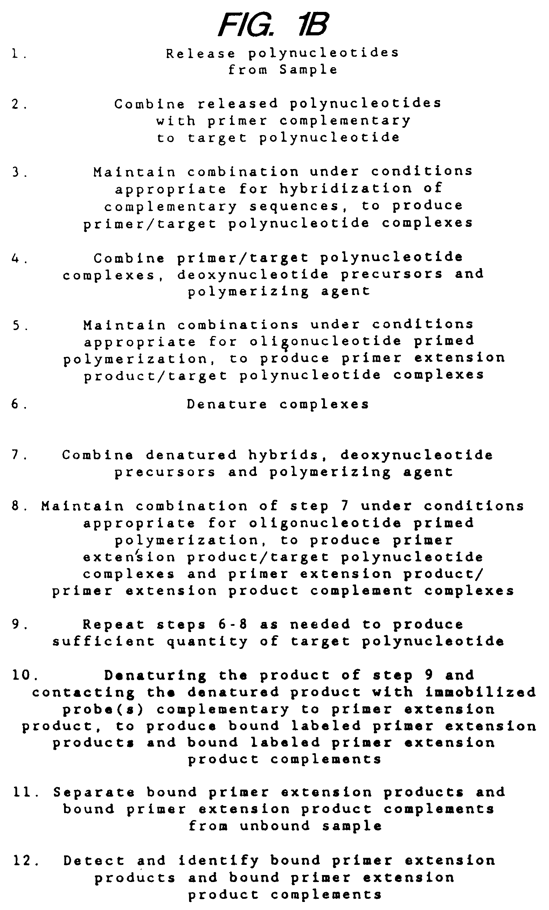

Brief Description of Figure 1

Figure 1 is a schematic representation of the method of the present invention. Figure 1A is an outline of the general strategy, by which RNA or DNA is amplified and detected. Figure 1B is a detailed representation of one embodiment of the present method.

Figure 2 is a schematic representation of the method of the present invention in detecting target organisms in a biologically rich sample.

Figure 3 is a schematic representation of the method of the present invention in detecting target organisms in a biologically poor sample.

Detailed Description of the Invention

The present invention is directed to a method for the detection in a sample of a polynucleotide target sequence or sequences whose presence is indicative of the presence in a sample of a specific organism or organisms (eg., bacteria or virus).

The present invention enables the detection of one or several different polynucleotide target sequences that are indicative of the presence of a single organism or several organisms in a given sample, such as a body fluid or appropriately processed tissue, water, food or beverage. For example, the detection in a biological sample, such as blood, of any bacterial organism, by the detection of one or more polynucleotide sequences present in the bacterial organism (referred to herein as target polynucleotides) can be carried out by the present method (e.g., as a means of diagnosing bacterial blood septicemia). This is accomplished by the rapid and repeated copying of one or more specific complements of the polynucleotide target sequences or first primer extension product (which is a copy of the polynucleotide target sequence) known to be indicative of the presence of certain organisms, while simultaneously incorporating a detection moiety into the primer extension product. The resulting "labeled" primer extension product is easily detected and its presence is indicative of the presence of the organism. Many clinical samples contain a quantity of pathogenic organisms too small to be detected by conventional methods (without the use of time-consuming culturing steps), and the present invention makes it possible to carry out the detection of smaller numbers of organisms than is possible with presently available methods.

The present method is represented schematically in Figure 1A and B. As shown, a sample to be analyzed is processed to render polynucleotide sequences present in the sample available for primer-extension (by releasing them from cells and, if necessary, rendering them single stranded). The resulting polynucleotides (referred to as available polynucleotides) include target polynucleotides (i.e., those to be detected in the sample), and other polynucleotides. Target polynucleotides can be DNA or RNA and some of the subsequent steps and reagents used will depend on which type of target is to be detected. For example, if the target polynucleotide is RNA, reverse transcription or a polymerase chain reaction using appropriate enzymes and labeled precursors can be carried out. This results in amplification of the target polynucleotide and production of labelled detectable target-like polynucleotides, which are DNA or RNA. If the target polynucleotide is DNA, an amplification method such as the polymerase chain reaction can be used with labelled precursors. This results in amplification of the target polynucleotide and production of labelled detectable target-like polynucleotides which are DNA. Subsequently, the labelled target-like polynucleotides are hybridized with one or more probes selected for their ability to hybridize to a known nucleotide sequence or sequences. Such probes can be, for example, generic probes (i.e., probes which hybridize to a class, group or genus of organisms) or specific probes (i.e., probes which detect a subset of a larger class, group or genus of organisms, such as a subclass or species). Hybridization is detected using known methods, selected on the basis of the type of label present on the target-like polynucleotides.

One embodiment of the present method is represented schematically in Figure 1B. As shown the polynucleotides released from the sample are combined with oligonucleotide primers, deoxyribonucleotide triphosphate precursors and a polymerizing agent, either simultaneously or sequentially. Oligonucleotide primers and/or deoxynucleotide triphosphate precursors are detectably labeled, with the result that products of the oligonucleotide primed replication are detectably labeled.

The oligonucleotide primers are complementary or substantially complementary to a region of the target polynucleotide. The resulting combination of available polynucleotides, oligonucleotide primers and deoxynucleotide triphosphate precursors is maintained under conditions appropriate for hybridization of complementary nucleotide sequences and oligonucleotide primed polymerization to occur. Initially, oligonucleotide primer/target polynucleotide complexes are formed, as a result of hybridization of complementary nucleotide sequences. Subsequently, a cycle of oligonucleotide primed polymerization is carried out, resulting in production of target polynucleotide/primer extension product complexes.

The complexes resulting from the first cycle of polymerization are denatured and combined with appropriate deoxynucleotide triphosphate precursors and a polymerizing agent. The combination is maintained under conditions appropriate for hybridization of complementary nucleotide sequences and oligonucleotide primed polymerization. This second (and subsequent) cycle of polymerization results in production of the two types of complexes produced in the first cycle and, in addition, primer extension product/primer extension product complement complexes. The primer extension product complements are substantially homologous to or homologous to the target polynucleotide. The resulting complexes are denatured and the cycle repeated as needed to produce a quantity of target polynucleotides sufficient for detection and/or quantitation.

The product of the cycles of oligonucleotide primed polymerization is a mixture of the complexes and other polynucleotide sequences which are not target sequences and are not part of a complex. Separation of the complexes from the sample (and, thus, from other polynucleotide sequences) is effected by separating the complexes into single stranded components and contacting the mixture with separation probes, which are complementary to a sequence present in the target polynucleotide or a sequence present in the labeled primer extension product and are immobilized on a solid support. They hybridize, respectively, to labeled primer extension product and to labeled primer extension product complement (both referred to herein as primer extension product). This results in production of a labeled primer extension product/separation probe-solid support complex (and, thus, solid support-bound target polynucleotides). The labeled primer extension product is detected using known methods. Its presence is indicative of the presence of the target polynucleotide.

The present method is useful for a wide variety of samples, such as body fluids and tissues, stool samples, food and water. In each case, polynucleotides present in the sample must be released and made available for hybridization with complementary nucleic acids and subsequent polymerization or amplification. Amplification can be carried out using known methods, such as the polymerase chain reaction, reverse transcription and the transcription amplification system. In the present method, a key advantage is the ability to detect one or more pathogens in a complex sample and to do so in a simpler, more efficient manner than is possible with other methods.

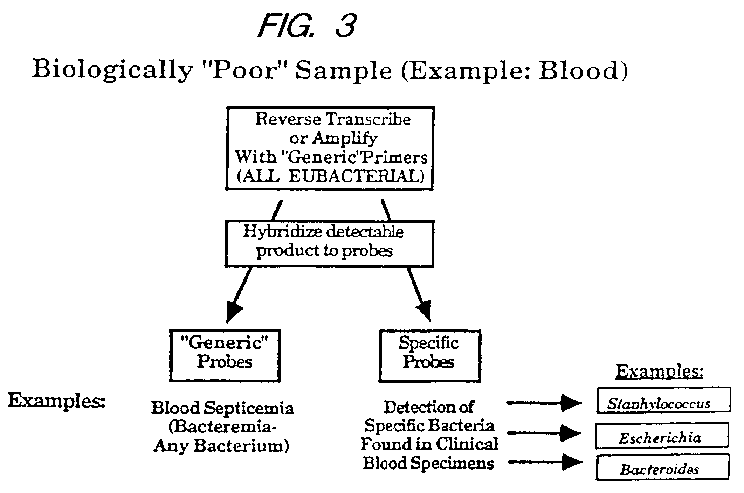

Two aspects of the present method particularly contribute to its simplicity and efficiency: the selection of target polynucleotide and the incorporation of detectable label into the replication product. That is, an important aspect of the present method is the target polynucleotide selected for polymerization or amplification In one embodiment, the target polynucleotide polymerized or amplified by the present method is a sequence, such as ribosomal RNA or the gene (DNA) encoding the ribosomal RNA (rRNA genes) which can be detected in a broad spectrum of pathogenic organisms. (See Figures 1A and 3). This embodiment is particularly useful in analyzing biologically "poor" samples (those in which organisms are present in low concentrations). Polynucleotides described in commonly owned U.S. patent application Serial No. 359,158, entitled Universal Eubacteria Nucleic Acid Probes and Methods and corresponding Patent Cooperation Treaty application PCT/WO90/15157 can be used for this purpose. Reverse transcription or amplification by oligonucleotide primed polymerization in which a "generic" primer (e.g., a primer for all eubacteria) is used can be followed by hybridization to generic separation probes or to specific separation probes. For example, generic separation probes can be used to diagnose blood septicemia or bacteremia by detecting the presence of any bacterium. Alternatively, specific separation probes, such as those specific for Staphylococcus, Escherichia or Bacteroides, can be used to detect bacteria in blood specimens.

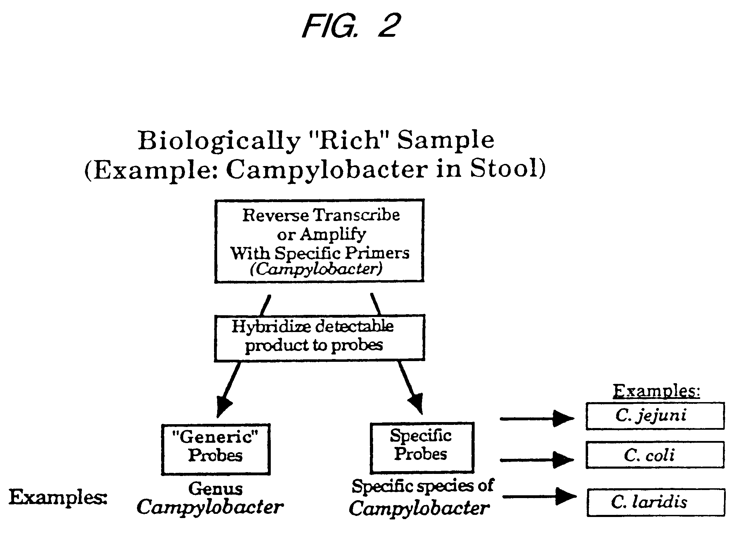

In a second embodiment, the target polynucleotide polymerized or amplified by the present method is a sequence present in a narrower (more limited) group of organisms, such as a genus of organisms. This embodiment, represented schematically in Figure 2, is particularly useful in analyzing biologically "rich" samples (those in which organisms are present in high concentrations). In this embodiment, for example, oligonucleotide primers specific for a bacterial genus (e.g., Campylobacter or Helicobacter) are used in oligonucleotide primed polymerization, which is followed by hybridization to generic separation probes or to specific separation probes. For example, separation probes which hybridize to a sequence or sequences present in all or most members of the Campylobacter genus (generic probes) can be used to detect the presence of any member(s) of the genus. Alternatively, separation probes which hybridize to a sequence or sequences present in specific species (e.g., C. jejuni, C. coli, C. laridis) of Campylobacter (specific probes) can be used to detect the specific species of interest.

A second important feature of the present invention is that as amplification or replication of the target polynucleotide occurs, a detectable moiety is incorporated into the amplification product. This is carried out by using one or more oligonucleotide primers which include a detectable moiety, oligonucleotide precursors which include a detectable moiety (e.g., $^{32}$P-deoxy ATP, fluorescent deoxyribonucleotides) or both. Use of such primers and/or precursors results in a detectably labeled product. After amplification and labelling of the target polynucleotide, the labeled product is contacted with a specific nucleic acid probe (separation probe) which is affixed to a solid support, under conditions appropriate for hybridization of complementary nucleic acid sequences. The labeled product becomes affixed to the solid support, is removed from the sample and detected using known means.

A variety of processes can be used for releasing nucleic acids from a sample to be analyzed. These processes include any physical or chemical method, such as sonication, bead-beaters, phenolextraction, or enzymatic lysis. Preferably, the nucleic acids are released by the method of dilutionlysis-centrifugation, well known in the art, followed by solubilization in a chaotropic solvent, such as guanidine thiocynanate.

In the initial cycle, the specific primer extension product, which is complementary to the polynucleotide target sequence is produced by using the polynucleotide containing the target sequence as a template. If the polynucleotide is double stranded, it is necessary to separate the strands of the polynucleotide before it can be used as a template. Separation can be effected either as a separate step or simultaneously with the synthesis of the primer extension products. Strand separation can be accomplished by any suitable denaturing method, including physical, chemical or enzymatic means. One physical method of separating the strands of nucleic acid involves heating the nucleic acid until it is completely denatured. Typical heat denaturation may involve temperature ranging from about 80 degrees to 105 centigrade for times ranging from about one to ten minutes. Strand separation may also be induced by bead reciprocating manipulation.

Any specific nucleic acid sequence and/or its complement (e.g., single stranded ribosomal DNAs or RNAs, such as 16S rDNA or rRNA of Escherichia coli) can be copied and labeled by the present method, as long as suitable primers are used. There are two key characteristics of the primers used. First, a sufficient number of bases at one end of the target sequence must be known in sufficient detail that oligonucleotide primers which will hybridize to the desired sequence can be produced. Second, the oligonucleotides primers must be of sufficient length that they remain hybridized under conditions used.

As used herein, in reference to primers and probes, the term oligonucleotide means any polynucleotide with generally at least 10 nucleotides, but fewer than 200 nucleotides.

"Primer" as that term is used herein, refers to an oligonucleotide probe which is capable of acting as a point of initiation of synthesis when used under conditions in which synthesis of a primer extension product, which is complementary to a polynucleotide sequence is induced (i.e., in the presence of precursors and a polymerizing agent).

The primers herein are selected to be "substantially complementary" to the different strands of each specific sequence to be amplified. This means that the primers must be sufficiently complementary to hybridize with their respective polynucleotide target sequences or primer extension products under the conditions used. Therefore, the primer sequence need not be exactly complementary to the target sequence or first primer extension product. For example, if an oligonucleotide primer is used, preferably 9 out of the 10 bases will be complementary to the target sequence or primer extension product.

The primers may be labeled or unlabeled. The unlabeled oligonucleotide primers may be prepared using one of several methods known to those skilled in the art. Primers may be prepared in such a manner that the label is introduced during manufacture or is modified after the primer is produced. For example, primers may be produced and then be labeled with any detectable moiety, such as a radioactive marker or fluorescent dye. The primers must be labeled in such a way as not to interfere with hybridization to the target sequence or with separation probe or subsequent primer extension. In addition, the label must be stable under primer extension procedures. For example, the label must be thermostable to survive the heating and cooling of the copying steps. In one embodiment, a fluorescein or rhodamine dye is attached by a short linker arm to the 5' end of the primer or to one of its purines or pyrimidine bases.

Suitable oligonucleotide primers include any eubacterial probes (e.g. Probes 919, 1638, 1660, 1739, and their complements as described in commonly assigned U.S.S.N. 359,158, Universal Eubacteria Nucleic Acid Probes and Methods and corresponding Patent Cooperation Treaty application PCT/WO90/15157), Campylobacter probes (e.g. probes and their complements, as described in U.S.S.N. 216,679), Escherichia coli probes (e.g. probes, and their complements, as described in U.S.S.N. 233,683, Probes for the Specific Detection of Escherichia coli and Shigella, the teachings of which are incorporated herein by reference) and Bacteroides probes (e.g. primers or probes which detect all bacteria, followed by Bacteriodes specific oligonucleotides).

If the original polynucleotide containing the target sequence to be replicated is single stranded, its complement is synthesized by adding one oligonucleotide primer. If an appropriate single primer is added, a primer extension product is synthesized in the presence of the primer, a polymerizing agent and appropriate precursors, as described above. The primer extension product will be partially complementary to a portion of the single stranded polynucleotide and will hybridize with the polynucleotide to form a duplex of unequal length strands. The duplex is then separated into single strands, as described above, to produce two single separated complementary strands.

When the complementary strands of the primer extension product and polynucleotide are separated, the original polynucleotide containing the target polynucleotide sequence or sequences and the first primer extension product are ready to be used for the synthesis of additional labeled complementary strands or new copies of the target sequence.

This synthesis can be performed using any suitable amplification or replication scheme. For example, polymerase chain reaction (PCR) or replication schemes utilizing reverse transcription can be used.

The unlabeled deoxyribonucleotide triphosphate precursors suitable for use in the present invention may be prepared by using methods known to those skilled in the art. Labeled precursors may be labeled with any detectable moiety, such as a radioactive marker or a fluorescent dye. The precursor must be labeled such that the label will not interfere with the primer extension procedure and the label used must be stable throughout the procedure. An example of a suitable labeled precursor is deoxyadenosine 5'-[$\alpha$-thio]-triphosphate labeled with $^{35}$S in the $\alpha$-thio position (dATP[$\alpha$-$^{35}$S]), available from New England Nuclear. "Precursor" as that term is used herein refers to deoxyribonucleotide triphosphates (designated G,A,T and C or analogues such as dUTP or dITP).

The deoxy-ribonucleotide triphosphate precursors are added to the sample mixture containing the primer extension/target complex in adequate amounts, and the resulting solution is heated to about 90°C to about 100°C, preferably 94°C, for about 1 to 4 minutes. After this heating period the solution is allowed to cool to from 37°C to 55°C centigrade, preferably 37°C. Once cooled, a polymerization agent is added, the mixture is maintained under conditions appropriate for oligonucleotide polymerization and amplification occurs. The synthesis reaction may occur at any temperature at which the polymerizing agent functions efficiently, generally not higher than 72°C. In those cases in which AMV reverse transcriptase is used, the temperature will generally not be higher than 55°C. The polymerizing agent may be any compound or system which will function to accomplish the synthesis of primer extension products, for example enzymes. Suitable enzymes for this purpose include DNA polymerases, reverse transcriptase, and other enzymes which will facilitate combination of the nucleotides in the proper manner to form primer extension products. Generally the synthesis will be initiated at the 3' end of each primer and proceed in the 5' direction along the template strand, until synthesis terminates, producing molecules of different lengths. There may be agents, however, which initiate synthesis at the 5' end and proceed in the other direction, using the same process as described above.

The newly synthesized primer extension product and its complementary polynucleotide target sequence form a double stranded molecule which is used in succeeding steps of the process. In the next step the strands of the double stranded molecule are separated using any procedures as described above to provide a single stranded molecule.

New primer extension product is synthesized on the single polynucleotide target sequence or the first primer extension product from the first cycle. Additional polymerizing agent, nucleotides and primers are added, if necessary for the reaction to proceed under conditions prescribed above. Again the synthesis will be initiated at one end of the oligonucleotide primers and will proceed along the single strands of the template to produce additional nucleic acid. After this step, half of the extension products will consist of the specific complement of the polynucleotide target sequence bounded by the one primer and the other half of the extension products will consist of the specific copy of the polynucleotide target sequence bounded by the one primer.

The steps of strand separation and extension product synthesis can be repeated as often as needed to produce the desired quantity of specific labeled sequences (complements and copies of the original polynucleotide target sequence). The amount of labeled primer extension product sequences produced will accumulate in a rapid fashion.

The present invention can be performed in a stepwise fashion where after each step new reagents are added, or simultaneously, where all reagents are added at the initial step, or partially stepwise and partially simultaneously, where fresh reagent is added after a given number of steps. If the method of strand separation used inactivates the polymerizating agent, as in the case of heat labile enzyme, then it is necessary to replenish the polymerizating agent after every strand separation step. The steps of this process can be repeated indefinitely, being limited only by the amount of primers, the polymerizating agent and precursors present. The number of cycles required to produce a detectable signal in sufficient quantity depends, for example, on the nature of the sample. For example, if the sample is relatively pure, with a high concentration of target sequences, then fewer cycles may be required.

Separation probes suitable for use in the present invention may be prepared by using a method similar to that which is used for preparing unlabeled oligonucleotide primers as explained above. The probes are chosen for their ability to hybridize to a specific target sequence of a known organism.

Matrices suitable as a solid support for immobilizing separation probes include nitrocellulose, nylon, polystyrene and magnetic beads. In one embodiment, separation probes are affixed to a nylon membrane by capillary blotting in high salt and subsequently treated with UV-irradiation (Khandjian, E., Biotechnology, 3:165-167 (1987).

The present invention will now be illustrated by the following examples, which are not intended to be limiting in any way.

Example 1 Clinical diagnosis of bacterial sepsis of blood

Blood is typically a biologically poor sample ("bacterially" poor). The following is a description of use of the present method with a biologically poor sample. A pair of oligonucleotides specific for hybridization to virtually all eubacteria such as Probe 1740 and the complement of Probe 1660 (described in commonly assigned USSN 359,158; Eubacterial Screens Patent Appl.) are used to enzymatically multiply a segment of the 16S (bacterial) ribosomal DNA, if present, in a sample of nucleic acid derived from a clinical blood specimen. Methods of dilution-lysis-centrifugation, followed by solubilization in a chaotropic solvent, such as quanidine thiocyanate. Alternatively, standard Methods, such as phenol extraction can be used to release RNA or DNA. The enzymatic multiplication requires a polymerase enzyme and deoxyribonucleotide triphosphates (designated G, A, T, and C) as precursors. One or more of these contains a detection moiety (for example, the A can be in the form of deoxy-$^{32}$P;ATP).

The product of this reaction is then hybridized to a filter membrane with an additional "all-bacterial" probe, such as Probe 1739 (described in commonly assigned USSN 359,158; Universal Eubacteria Nucleic Acid Probes and Methods and corresponding Patent Cooperation Treaty application PCT/WO90/15157). If any bacterial organism is present in the original sample, the 16S rDNA segment is replicated and $^{32}$P labeled. This material is detectable as nucleic acid hybridized to the filter bound Probe 1739. Filter bound detection moiety, in this case radioactivity, is indicative of the presence of bacterial rDNA, and thus leads to a positive diagnosis of bacterial septicemia.

In a specific test carried out on a blood sample obtained from a patient suspected of having blood septicemia by methods of dilution-lysis-centrifugation, followed by solubilization in a chaotropic solvent (e.g., guanidine thiocyanate), a pair of oligonucleotide primers (each at 0.01 mg/ml in water) specific for all eubacteria is used. Primer/Probe 1739 and a probe complementary to Primer/Probe 1638 (see commonly assigned U.S.S.N. 359,158, Universal Eubacteria Nucleic Acid Probes and Methods and corresponding Patent Cooperation Treaty application PCT/WO90/15157, the teachings of which are incorporated herein by reference) are added to the treated sample.

If primer extension and pseudo-target labeling are done with reverse transcriptase, either AMV or MMLV reverse transcriptase is added, along with a suitable buffer, and precursors (deoxy-nucleotide triphosphates or dNTPs), labeled and unlabeled. In this case, Primer/Probe 1739 is used. The solution reaction (containing buffer, enzyme, dNTPs, labeled dNTPs, sample RNA and Primer/Probe 1739) is incubated at a temperature of between 37°C and 50°C. This results in incorporation of the labeled dNTPs into a polynucleotide complementary to the 16S rRNA of the bacterium being detected (if present in the sample).

If primer extension and pseudo-target labeling are done with the polymerase chain reaction, the reaction mixture consists of: buffer, an enzyme such as Taq, Vent or other similar thermostable polymerase, dNTPs, labelled dNTPs, sample DNA, Primer/Probe 1739 and Primer/Probe 1638. This combination is thermally cycled among temperatures of 94°C, 42°C and 72°C, resulting in copies of a fragment of the 16S rDNA of the bacterium being detected (if present in the sample).

In addition, labeled target polynucleotide can be produced using a combination of the enzyme activities of reverse transcriptase and thermostable polymerase.

Example 2 Specific identification of a bacterial sepsis-causing organism

The detectable target-like nucleic acid produced in Example 1 is further examined following the positive diagnosis of that example. Specific candidate species probes, which hybridize to this in vitro labeled bacterial rDNA sequence, are used. For example, Probe 1660 (described in commonly assigned USSN 359,158, Universal Eubacteria Nucleic Acid Probes and Methods and corresponding Patent Cooperation Treaty application PCT/WO90/15157)) is specific for all known eubacterial sequences. Hybridization of Probe 1660 (or a probe sequence complementary to Probe 1660) to a labeled product of the chain elongation described in Example 1 results in trapping of detectable signal onto the solid support. Detection of signal (e.g., light, radioactivity, color) associated with the solid support confirms a positive diagnosis of bacterial septicemia. Alternatively, Probe 787 (described in commonly owned U.S.S.N. 233,683, Probes for the Specific Detection of Escherichia coli and Shigella and corresponding application EP 89308413.7 the teachings of which are incorporated herein by reference) can be used as a separation probe to provide a positive identification of septicemia caused by E. coli. E. coli is a major agent of bacterial septicemia and, thus, the utility of this diagnostic step is obvious.

It is also possible to detect disseminated Neisseria gonorrhoeae infection by using Probe 919 (described in U.S.S.N. 356,155, the teachings of which are incorporated herein by reference) in the separation probe. Hybridization of chain elongation product to Probe 919 or sequence complementary to it would provide positive identification of such an infection.

Note that this specific identification of the species causing the septic infection is done with the same material initially prepared from the enzymatic amplification/labelling. In practice, several different procedures of diagnosis and identification can be followed:

(1) From the initial amplification/labelling the presumptive bacterial material is simultaneously hybridized to probes for (a) any bacterium, (b) E. coli, (c) Staphylococci, (d) Bacteroides species, (e) etc.

(2) Specific tests, such as (b) through (e), above, are performed only after a positive result from Example 1.

In either case, the initial clinical sample need only be processed once, in order to enable a variety of specific and generic tests to be performed.

Example 3 Simultaneous diagnosis of Campylobacter infection and identification of the specific pathogenic species

Unlike blood, samples such as stools are bacterially "rich". Any type of nucleic acid template copying lacking specificity will merely over-represent the most prevalent species (in stools, for example, Bacteroides, Fusobacteria, and members of a few other highly prevalent genera). In order to identify the presence of a minor bacterial component, primer extension must be mediated by oligonucleotides with greater specificity than those used in Example 1. In this example, primers are specific for the genus Campylobacter. Subsequent hybridization, as in Example 2, is to probes specific for specific Campylobacter species, for example, C. jejuni, C. coli, C. laridis, etc.

Equivalents

Those skilled in the art will recognize or be able to ascertain using no more than routine experimentation, many equivalents to the specific embodiments of the invention described specifically herein. Such equivalents are intended to be encompassed in the scope of the following claims.

**Claims**

1. A method of detecting, in a sample, a polynucleotide target sequence, comprising the steps of:

   a) releasing polynucleotides present in the sample, thereby producing released polynucleotides,

   b) combining released polynucleotides with:

   1) at least one oligonucleotide primer which is substantially complementary to the target nucleotide sequence;

   2) deoxyribonucleotide triphosphate precursors and

   3) a polymerizing agent, at least one primer or at least one precursor being detectably labeled,

   c) maintaining the combination formed in (b) under conditions appropriate for hybridization of substantially complementary nucleotide sequences and oligonucleotide primed polymerization, for sufficient time to complete one cycle of oligonucleotide primed polymerization, thereby forming labeled primer extension product/target polynucleotide complexes;

   d) denaturing the labeled complexes, thereby producing denatured complexes;

   e) combining denatured complexes, deoxyribonucleotide triphosphates and a polymerizing agent;

   f) maintaining the combination produced in (e) under conditions appropriate for hybridization of substantially complementary nucleotide sequences and oligonucleotide primed polymerization, thereby forming labeled primer extension product/target polynucleotide complexes and labeled primer extension product/primer extension product complement complexes;

   g) repeating steps (d), (e) and (f) to produce a sufficient quantity of labeled primer extension products and labeled primer extension product complements sufficient for detection;

   h) denaturing the product of step (g);

   i) contacting the product of step (h) with a solid support having affixed thereto at least one separation probe which is sufficiently complementary to the labeled primer extension products to hybridize to the labeled primer extension products and the labeled primer extension product complements under the conditions used, thereby forming bound labeled primer extension products and bound labeled primer extension product complements;

   j) separating the bound labeled primer extension products and bound labeled primer extension product complements from unbound sample; and

   k) detecting the bound labeled primer extension products and bound labeled primer extension product complements, the presence of which is indicative of the presence in the sample of the target polynucleotide.

2. The method of Claim 1, wherein in step (f), more than one separation probe is used.

3. The method of Claim 1, wherein the target polynucleotide is a single stranded ribosomal RNA.

4. The method of Claim 3, wherein the target polynucleotide is single stranded ribosomal DNA.

5. The method of Claim 4, wherein the single stranded ribosomal DNA is a 16S rDNA of Escherichia coli.

**6.** The method of Claim 3, wherein the polymerizing agent is reverse transcriptase.

**7.** A method of detecting, in a sample, a target ribosomal RNA, comprising the steps of:

a) releasing polynucleotides present in the sample, thereby producing a sample containing released polynucleotides;

b) combining the sample containing released polynucleotides with:

1) at least one oligonucleotide primer which is substantially complementary to the target ribosomal RNA;

2) deoxyribonucleotide triphosphate precursors, and

3) a polymerizing agent, at least one primer or at least one precursor being detectably labeled;

c) maintaining the combination formed in (b) under conditions appropriate for hybridization of substantially complementary nucleotide sequences and oligonucleotide primed polymerization, for sufficient time sufficient to complete one cycle of oligonucleotide primed polymerization, thereby forming labeled primer extension product/target polynucleotide complexes;

d) denaturing the labeled complexes, thereby producing labeled primer extent product and target polynucleotides;

e) combining the product of (d), deoxyribonucleotide triphosphates and a polymerizing agent;

f) maintaining the combination produced in (e) under conditions appropriate for hybridization of substantially complementary nucleotide sequences and oligonucleotide primed polymerization, thereby forming labeled primer extension product/target polynucleotide complexes and labeled primer extension product/labeled primer extension product complement complexes;

g) denaturing the complexes formed in (f), thereby producing labeled primer extension product, target polynucleotides and labeled primer extension product complements;

h) repeating steps (d), (e) and (f) and (g) to produce a sufficient quantity of labeled primer extension products and labeled primer extension product complements sufficient for detection;

i) denaturing the product of step (h);

j) contacting the product of step (i) with a solid support having affixed thereto at least one separation probe which is sufficiently complementary to the labeled primer extension products to hybridize to the labeled primer extension products and the labeled primer extension product complements under the conditions used, thereby forming bound labeled primer extension products;

k) separating the bound labeled primer extension products and the bound labeled primer extension product complements from the unbound sample; and

l) detecting the bound labeled primer extension products and the bound labeled primer extension product complements, the presence of which is indicative of the presence in the sample of the target ribosomal RNA.

**8.** The method of Claim 7 , wherein more than one separation probe is used.

**9.** The method of Claim 8 , wherein the ribosomal RNA is 16S rRNA of Escherichia coli.

**10.** The method of Claim 7, wherein the polymerizing agent of step (b) is reverse transcriptase.

**11.** The method of Claim 3 or claim 7, wherein the oligonucleotide primer of step (b) is a eubacterial probe.

**12.** The method of Claim 11, wherein the eubacterial probe is selected from the group consisting of Probe 919, Probe 1660, Probe 1739, and their complements.

**13.** The method of Claim 3 or claim 7, wherein the oligonucleotide primer of step (b) is a Campylobacter probe.

**14.** The method of Claim 13, wherein the Campylobacter probe is selected from the group consisting of probes specific for Campylobacter jejuni, Campylobacter coli and Campylobacter laridis.

**15.** The method of Claim 3 or claim 7, wherein the oligonucleotide primer of step (b) is selected from the group consisting of: Escherichia coli probes, Staphylococcus probes and Bacteroides fragilis probes.

*FIG. 1A*

```
                    ┌─────────────────┐
                    │ CLINICAL SAMPLE │
                    └─────────────────┘
                             │
                             ▼
                   ┌───────────────────┐
                   │ PROCESS TO YIELD  │
                   │   NUCLEIC ACIDS   │
                   └───────────────────┘
```

RNA                                    DNA

```
  ┌──────────────────┐        ┌──────────────────┐
  │     REVERSE      │        │   POLYMERASE     │
  │   TRANSCRIBE     │        │ CHAIN REACTION   │
  │  INCORPORATING   │        │  INCORPORATING   │
  │    DETECTION     │        │    DETECTION     │
  └──────────────────┘        └──────────────────┘
              │                        │
              ▼                        ▼
            ┌──────────────────────────┐
            │       "LABELLED"         │
            │       DETECTABLE         │
            │      TARGET-LIKE         │
            │    POLYNUCLEOTIDE        │
            └──────────────────────────┘
                        │
                        ▼
         ┌────────────────────────────────┐
         │        HYBRIDIZE TO            │
         │     PANEL OF KNOWN             │
         │     (CHARACTERIZED)            │
         │         PROBES                 │
         │ (for example: PROBES A-F)      │
         └────────────────────────────────┘
```

A    B    C    D    E    F

Results of hybridization
to probe panel:     -    -    +    -    +    -

# FIG. 1B

1.   Release polynucleotides
     from Sample

2.   Combine released polynucleotides
     with primer complementary
     to target polynucleotide

3.   Maintain combination under conditions
     appropriate for hybridization of
     complementary sequences, to produce
     primer/target polynucleotide complexes

4.   Combine primer/target polynucleotide
     complexes, deoxynucleotide precursors and
     polymerizing agent

5.   Maintain combinations under conditions
     appropriate for oligonucleotide primed
     polymerization, to produce primer extension
     product/target polynucleotide complexes

6.   Denature complexes

7.   Combine denatured hybrids, deoxynucleotide
     precursors and polymerizing agent

8.   Maintain combination of step 7 under conditions
     appropriate for oligonucleotide primed
     polymerization, to produce primer
     extension product/target polynucleotide
     complexes and primer extension product/
     primer extension product complement complexes

9.   Repeat steps 6-8 as needed to produce
     sufficient quantity of target polynucleotide

10.  Denaturing the product of step 9 and
     contacting the denatured product with immobilized
     probe(s) complementary to primer extension
     product, to produce bound labeled primer extension
     products and bound labeled primer extension
     product complements

11.  Separate bound primer extension products and
     bound primer extension product complements
     from unbound sample

12.  Detect and identify bound primer extension
     products and bound primer extension
     product complements

## FIG. 2

**Biologically "Rich" Sample**
**(Example: Campylobacter in Stool)**

Reverse Transcribe
or Amplify
With Specific Primers
*(Campylobacter)*

Hybridize detectable
product to probes

"Generic"
Probes

Specific
Probes

Examples:    Genus
*Campylobacter*

Specific species of
*Campylobacter*

Examples:

C. *jejuni*

C. *coli*

C. *laridis*

EP 0 517 361 A1

## FIG. 3

### Biologically "Poor" Sample (Example: Blood)

Reverse Transcribe
or Amplify
With "Generic"Primers
(ALL EUBACTERIAL)

Hybridize detectable
product to probes

"Generic"
Probes

Specific
Probes

Examples:

Examples:

Blood Septicemia
(Bacteremia-
Any Bacterium)

Detection of
Specific Bacteria
Found in Clinical
Blood Specimens

Staphylococcus

Escherichia

Bacteroides

| DOCUMENTS CONSIDERED TO BE RELEVANT | | | EP 92303861.6 | |
|---|---|---|---|---|
| Category | Citation of document with indication, where appropriate, of relevant passages | Relevant to claim | CLASSIFICATION OF THE APPLICATION (Int. Cl.5) | |
| A | EP - A - 0 395 292 (BARRY et al.) * Abstract; claims * | 1-4,7 | C 12 Q 1/68 //(C 12 Q 1/68 C 12 R 1:19) (C 12 Q 1/68 C 12 R 1:44) (C 12 Q 1/68 C 12 R 1:01) | |
| A | WO - A - 89/09 284 (UNIVERSITY OF IOWA RESEARCH FOUNDATION) * Abstract; claims * | 1-4,7 | | |
| A | WO - A - 89/09 282 (HOLMES et al.) * Abstract; claims * | 1-4,7 | | |
| A | EP - A - 0 329 822 (CANGENE CORPORATION) * Abstract; claims * | 1-4,7 | | |
| P,A | PATENT ABSTRACTS OF JAPAN, unexamined applications, C section, vol. 15. no. 302, August 2, 1991 THE PATENT OFFICE JAPANESE GOVERNMENT page 114 C 855 * Kokai-no. 3-112 497 (SHIMADZU CORPORATION) * | 1-4;7 | TECHNICAL FIELDS SEARCHED (Int. Cl.5) C 12 Q | |
| P,A | PATENT ABSTRACTS OF JAPAN, unexamined applications, C section, vol. 16, no. 66, February 19, 1992 THE PATENT OFFICE JAPANESE GOVERNMENT page 103 C 912 * Kokai-no. 3-262 500 (NIPPON SHOJI K.K) * | 1-4,7 | | |
| D,A | WO - A - 90/15 157 (GENE-TRAK SYSTEMS) * Abstract; claims * | 1-4,7 | | |

The present search report has been drawn up for all claims

| Place of search | Date of completion of the search | Examiner |
|---|---|---|
| VIENNA | 17-09-1992 | SCHNASS |